# EUROPEAN PATENT APPLICATION

(11) **EP 1 191 335 A2**
(43) Date of publication of application: **27.03.2002**
(21) Application number: 01121773.4
(22) Date of filing: 19.09.2001
(51) Int. Cl.: G01N 33/36

(54) **Device for measuring the count of a ribbon of fibers**

(30) Priority: 22.09.2000 IT PD000222
(71) Applicant: Tomsic s.r.l., 34070 Savogna D'Isonzo (Gorizia) (IT)
(72) Inventor: Tomsic, Silvan, 34070 Savogna D'Isonzo (Gorizia) (IT)
(74) Representative: Modiano, Guido, Dr.-Ing.

(57) **Abstract**

A device for measuring the count of a ribbon of fibers, comprising a guiding funnel (11) for a ribbon (12) of fibers that tapers along the direction in which the ribbon moves and has, at a portion (14) that has the smallest cross-section, a pressure port (15) connected to an external unit that comprises means adapted to detect the air pressure value.

## Description

The present invention relates to a device for measuring the count of a ribbon of fibers.

These devices are normally used in spinning systems to monitor whether the count of the ribbon of fibers, i.e., its mass per unit length, varies from the intended value within industrially acceptable limits.

The measurement devices currently used are substantially constituted by rollers that compress the ribbons and whose relative movement is measured in order to determine the count of the ribbon, which is a function of such movement.

Conventional devices are particularly precise, but have a complicated geometry caused in particular by the number of transmission elements required to transmit the motion to the two rollers.

The aim of the present invention is to provide a device for measuring the count of a ribbon of fibers that has a simple structure.

Within this aim, an object of the invention is to provide a device that is smaller than conventional devices.

Another object is to provide a measurement device that is reliable and precise.

A further object is to provide a device that can be manufactured at low cost.

A still further object is to provide a device by using conventional equipment and technologies, which can also be applied to already-installed systems.

This aim and these and other objects which will become better apparent hereinafter are achieved by a device for measuring the count of a ribbon of fibers, characterized in that it comprises a guiding funnel for a ribbon of fibers that tapers along the direction in which the ribbon moves and has, at the portion that has the smallest cross-section, a pressure port connected to an external unit that comprises means adapted to detect the air pressure value.

Further characteristics and advantages of the invention will become better apparent from the description of a preferred but not exclusive embodiment thereof, illustrated only by way of non-limitative example in the accompanying drawings, wherein:
Figure 1 is a functional block diagram of the device according to the invention;
Figure 2 is a sectional view of a portion of the device according to the invention;

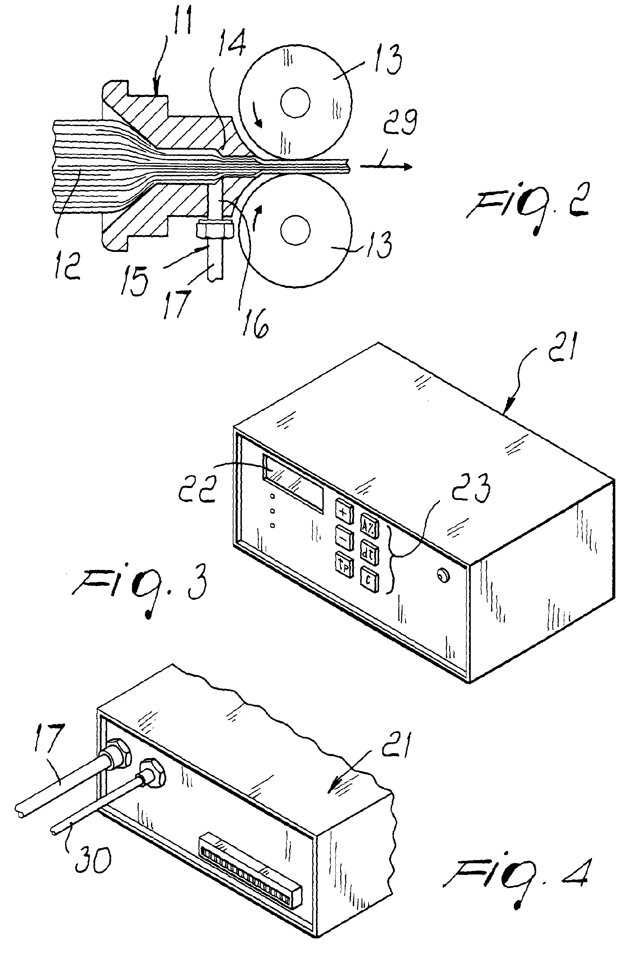

Figures 3 and 4 are views, from two different viewpoints, of a component of the device according to the preceding figures.

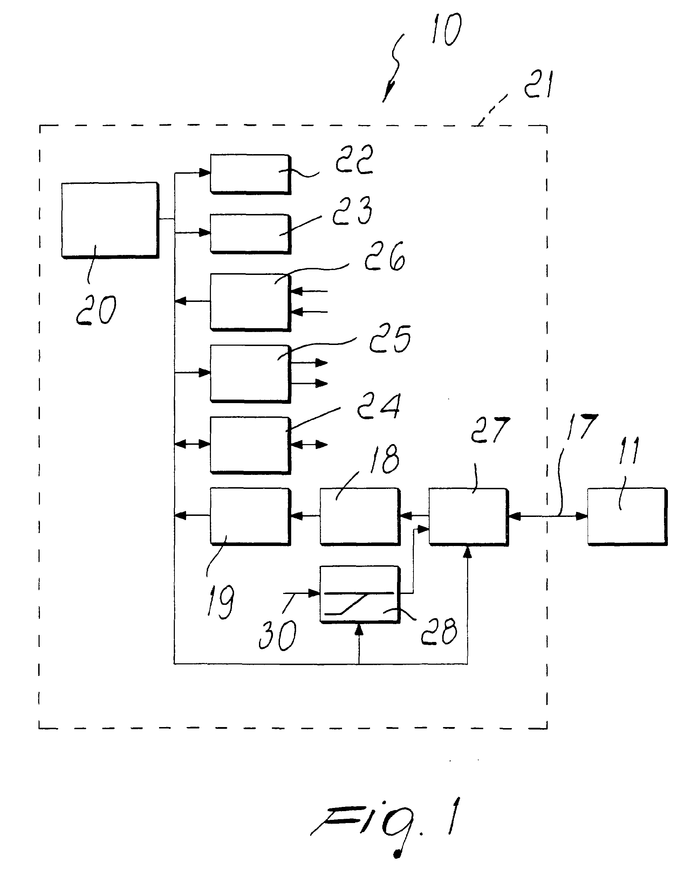

With reference to Figure 1, a device for measuring the count of a ribbon of fibers according to the invention is generally designated by the reference numeral 10.

The device 10 comprises a guiding funnel 11, in which a ribbon of fibers 12 slides by virtue of traction means, which are generally designated by the reference numeral 13 and are constituted by two cylinders, designated by the same reference numeral, which are arranged mutually opposite with respect to the motion of the ribbon of fibers 12.

The funnel 11 tapers in the direction of motion, designated by the arrow 29, of the ribbon of fibers 12 and has, at a portion 14 that has a smaller cross-section, a pressure port generally designated by the reference numeral 15.

The pressure port 15 is constituted in practice by a radial duct 16 that is formed in the funnel 11 and is connected by means of a hose 17 to a sensor, schematically shown in Figure 1 and designated by the reference numeral 18, which will be described in greater detail hereinafter.

In practice, upstream of the funnel 11, between one fiber and the next of the ribbon of fibers 12 there is air; by passing through the funnel 11, the ribbon of fibers 12 is compressed, generating inside the funnel 11 a pressure that reaches its maximum value at the very portion 14 of the funnel 11, which has a smaller cross-section.

Such pressure value is detected by the sensor 18 by virtue of the pressure port 15; in this case, said sensor is a transducer adapted to generate an electrical signal that corresponds to the detected pressure value.

By means of an analog/digital converter 19, the electrical signal is converted into a signal that can be detected by a processing and control unit 20 which, together with the sensor 18, is contained in an external unit 21 connected to the funnel 11 by means of the hose 17.

A display 22 for displaying the processed data, a keyboard 23 for interfacing with the user, and a serial interface 24 for connection to electronic computers are provided on the external unit 21 and are connected and therefore controlled by the processing and control unit 20.

The external unit 21 further comprises means 25 capable of stopping the spinning machine with which the device 10 is associated and/or alarm signaling means.

The device 10 can also comprise an interface 26 for digital inputs connected to the processing and control unit 20; such inputs can be constituted by sensors for detecting the speed of the spinning machine and/or sensors adapted to detect whether the spinning machine is operating or not.

During the measurement process, the ribbon of fibers 12 tends to dirty the measurement hose 17, and for this reason the device 10 is provided with cleaning means for the pressure port 15.

The cleaning means are constituted by two valves, namely a safety valve 27 and a cleaning valve 28, shown schematically in Figure 1 but in any case of the known type, controlled by the processing and control unit 20 and contained in the external unit 21.

In particular, the safety valve 27 is interposed on the hose 17 between the sensor 18 and the funnel 11.

The safety valve 27 is further connected to the cleaning valve 28, which is in turn connected to a compressed air circuit, designated by the reference numeral 30, in this case advantageously at a pressure of 4 bar.

During normal operation, the safety valve 27 is closed toward the cleaning valve 28 and therefore the sensor 18 detects the pressure inside the funnel 11 through the pressure port 15.

At adjustable intervals, the processing and control unit 20 temporarily suspends pressure measurement, closing the safety valve 27 toward the sensor 18 and opening its connection to the cleaning valve 28, which in turn opens, allowing pressurized air to enter and flow from the hose 17 toward the measurement funnel 11.

The air thus flows from the external unit 21 toward the funnel 11, pushing any dirt into the ribbon of fibers 12 which, by virtue of its normal motion, tends to carry it away.

As regards the overall operation of the device 10, the pressure that occurs at the portion 14 of the funnel 11 is detected and converted into a digital signal by means of the sensor 18 and the converter 19 and is managed by the processing and control unit 20.

The processing and control unit 20 converts the signal into a percentage variation of the count of the ribbon of fibers with respect to a nominal value.

The percentage value is displayed on the display 22 of the external unit 21.

When the percentage value exceeds the set limits, the device 10 stops the spinning machine with which it is associated, indicating the variation of the count at that precise point in time.

As mentioned, since the fibers tend to dirty the measurement hose 17, the processing and control unit 20 operates the cleaning means at adjustable intervals, temporarily suspending measurement and introducing compressed air in said hose 17 toward the funnel 11.

In practice it has been observed that the present invention has achieved its intended aim and objects.

A device for measuring the count of a ribbon of fibers has in fact been provided which has a simple structure and compact dimensions with respect to the prior art.

The measurement system is digital and therefore extremely precise and reliable.

It is possible to use funnels of different sizes according to the type of ribbon.

It is also evident that the device can be used in all spinning machines in which there are funnels that can be replaced with a funnel used in a device according to the invention.

Advantageously, it is possible to provide the funnels already present in spinning machines with a pressure port to be connected to an external unit as in the invention.

Finally, optimization of the entire spinning process is achieved by providing precise and reliable control over the count of the ribbon.

The present invention is susceptible of numerous modifications and variations, all of which are within the scope of the inventive concept.

The technical details may be replaced with other technically equivalent elements.

The materials and the dimensions may be any according to requirements.

The disclosures in Italian Patent Application No. PD2000A000222 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device (10) for measuring the count of a ribbon of fibers, **characterized in that** it comprises a guiding funnel (11) for a ribbon (12) of fibers that tapers along the direction in which the ribbon moves and has, at the portion (14) that has the smallest cross-section, a pressure port (15) connected to an external unit (21) that comprises means (18) adapted to detect the air pressure value.

2. The device according to claim 1, **characterized in that** said means adapted to detect the value of the air pressure comprise a transducer (18) that is connected to said pressure port (15) and is adapted to generate a corresponding electrical signal that can be acquired, by interposing an analog/digital converter (19), by a processing and control unit (20).

3. The device according to claim 1, **characterized in that** said pressure port (15) is constituted by a radial duct (16) formed in said funnel (11) and connected by means of a hose (17) to said external unit (21).

4. The device according to claim 3, **characterized in that** it comprises means (27,28) for cleaning said pressure port (15).

5. The device according to claim 4, **characterized in that** said cleaning means (27,28) comprise a safety valve (27), which is interposed between said funnel (11) and the transducer (18) on the hose (17) of the pressure port (15), and a cleaning valve (28), which is connected to said safety valve and to a circuit of compressed air adapted to flow from said cleaning and safety valves toward the funnel.

6. The device according to one or more of the preceding claims, **characterized in that** said external unit (21) comprises a display (22) for displaying the acquired data connected to said processing and control unit (20).

7. The device according to one or more of the preceding claims, **characterized in that** said external unit (21) comprises a keyboard (23) for interfacing with the user.

8. The device according to one or more of the preceding claims, **characterized in that** said external unit (21) comprises actuation means (25) for stopping a spinning machine with which said device is to be associated and/or alarm signaling means, said means being connected to said processing and control unit (20).

9. The device according to one or more of the preceding claims, **characterized in that** said processing and control unit (20) is connected to an electronic computer.

10. The device according to one or more of the preceding claims, **characterized in that** said external unit (21) comprises an interface (26) for digital inputs that is connected to said processing and control unit (20).

11. The device according to claim 1, **characterized in that** it comprises traction means arranged mutually opposite with respect to the direction of motion of the ribbon of fibers.

12. The device according to claim 11, **characterized in that** said traction means comprise two cylinders.
